(19) Europäisches Patentamt
European Patent Office
Office européen des brevets

(11) Publication number: **0 460 683 A2**

# (12) EUROPEAN PATENT APPLICATION

(21) Application number: **91109319.3**

(51) Int. Cl.[5]: **A61K 7/00**, A61K 7/06

(22) Date of filing: **06.06.91**

(30) Priority: **07.06.90 US 534801**

(43) Date of publication of application:
**11.12.91 Bulletin 91/50**

(84) Designated Contracting States:
**DE FR GB**

(71) Applicant: **DOW CORNING CORPORATION**
**P.O. Box 1767**
**Midland Michigan 48686-0994(US)**

(72) Inventor: **Halloran, Daniel Joseph**
**3825 Pfeiffer Court**
**Midland, Michigan(US)**
Inventor: **Kasprzak, Kenneth Alfred**
**3382 Bramble Drive**
**Saginaw, Michigan(US)**
Inventor: **Krzysik, Duane G.**
**5517 Woodview Pass**
**Midland, Michigan(US)**
Inventor: **Lake, Norman Eugene**
**2162 Dublin Road**
**Midland, Michigan(US)**

(74) Representative: **Spott, Gottfried, Dr. et al**
**Spott Weinmiller & Partner**
**Sendlinger-Tor-Platz 11**
**W-8000 München 2(DE)**

## (54) Silicone mechanical emulsions for hair care.

(57) A method is disclosed for treating hair by applying to the hair a formulation including at least one organosilicon compound and at least one additive selected from the group consisting of surfactants, conditioners, thickeners, plasticizers, fragrances, propellants and preservatives. The improvement relates to the step of incorporating the organosilicon compound into the formulation in the form of a mechanically prepared emulsion, with the particle size of the organosilicon compound in the emulsion being less than about 350 nanometers. A composition is also described.

EP 0 460 683 A2

This invention relates to a silicone mechanical emulsion for hair care applications. More particularly, the invention is directed to mechanically prepared silicone emulsions, in contrast to silicone emulsions prepared by emulsion polymerization and containing at least one organosilicon compound which is substantive to hair and which possesses a high viscosity and high molecular weight.

Silicone emulsions are stable mixtures of an organosilicon compound and water which are held together by means of a surfactant often referred to as an emulsifying agent. Such emulsions can be produced by either emulsion polymerization techniques or mechanically. There is a fundamental difference between emulsions produced by emulsion polymerization and emulsion produced mechanically. In emulsion polymerization, for example, cyclic organosilicon compounds of the formula $(Me_2SiO)_x$ are emulsified in water by adding a polymerization catalyst and heating the emulsion. Stable emulsions of high viscosity and high molecular weight can be obtained by this method containing organosilicon fluids of the order of magnitude of from about sixty to one hundred-thousand centistokes measured at 25° C. Apparatus for producing emulsions by emulsion polymerization is complex requiring a mixing tank, a homogenizer, a polymerization vessel and a neutralization tank. In contrast, mechanically produced silicone emulsions are produced with much simplified equipment requiring only a mixing tank and a colloid mill. Such mechanical silicone emulsions require merely the mixing of water and a surfactant with an organosilicon compound of high viscosity and high molecular weight, followed by shearing of the mixture.

The prior art is replete with patents relating to silicone emulsions produced by emulsion polymerization. The prior art is also replete with patents relating to silicone emulsions produced mechanically. However, few patents are directed to the concept of using mechanical emulsions of organosilicon compounds in hair care applications.

The present invention, therefore, can be distinguished from the prior art as enumerated above in that the hair care emulsions of the present invention are mechanically produced and contain organosilicon compounds of significantly higher viscosity and higher molecular weight than the hair care formulations heretofore known in the art.

This invention is directed to both a composition and a method of treating hair by applying to the hair a formulation including at least one organosilicon compound and at least one additive selected from the group consisting of surfactants, conditioners, thickeners, plasticizers, fragrances, propellants and preservatives. The improvement relates to the step of incorporating the organosilicon compound into the formulation in the form of a mechanically prepared emulsion. The particle size of the organosilicon compound in the emulsion is about 350 nanometers, preferably less than about three hundred nanometers.

In some of the more preferred embodiments of the present invention, the organosilicon compound may be a high viscosity polysiloxane, a high molecular weight polysiloxane, a functional polysiloxane or a bimodal polysiloxane. Where the organosilicon compound is a polysiloxane, it should have a viscosity of at least 50,000 centistokes measured at 25° C. Where the organosilicon compound is a functional polysiloxane, it may have a functional group such as carboxy, carboxyalkyl, haloalkyl, glycol, acrylamide, acrylate, acryloxy, vinyl or amino groups.

One particular embodiment of the present invention is directed to the use of an organosilicon composition which is a bi-modal polysiloxane and where the bi-modal polysiloxane is a mixture of a nonvolatile polydimethylsiloxane and a volatile or low viscosity polydimethylsiloxane. In this embodiment, the nonvolatile polydimethylsiloxane should have a viscosity of at least 50,000 centistokes measured at 25° C. and the volatile polydimethylsiloxane is a polydimethylcyclosiloxane of the formula $(CH_3)_2SiO_x$ in which x is an integer of from three to eight. The polydimethylcyclosiloxane has a viscosity measured at 25° C. of less than about one centistoke. The most preferred polydimethylcyclosiloxane is a mixture of octamethylcyclotetrasiloxane and decamethylcyclopentasiloxane.

The emulsion is prepared by combining the polysiloxane, water and a primary nonionic surfactant having an HLB value of 15-19 to form a first mixture, adding to the first mixture a co-surfactant selected from the group consisting of nonionic, cationic and anionic surfactants having an HLB value of 1.8-15 to form a second mixture and mixing the second mixture at a temperature of about 40° C. until the particle size of the polysiloxane in the emulsion is less than about three hundred nanometers. The co-surfactant is most preferably a nonionic surfactant having an HLB value of 1.8-15.

These and other features, objects and advantages, of the herein described present invention will become more apparent from a consideration of the following detailed description thereof.

The cosmetics and toiletry industry has produced a wide range of grooming aids for use by men and women. Among the myriad of such products are shampoos to clean the hair and scalp, hair rinses, conditioners, dressings, sprays, wave sets, coloring and bleaching preparations, permanent waves and hair straightening compositions. Cleanliness of hair and scalp are important personal grooming criteria. Extensively soiled hair takes on a lackluster appearance and becomes oily and gritty to the touch. The shampoo

must foam quickly and copiously and rinse out thoroughly leaving the hair with a fresh clean scent, soft, shiny, lustrous, full bodied and in a manageable state. While most shampoos are clear liquids and opaque lotions, they are available as clear gels, cream pastes and aerosols. Shampoos contain one or more cleaning agents such as anionic, nonionic, amphoteric or ampholytic and cationic surfactants; and various additives including viscosity control agents, opacifiers, conditioners, preservatives and fragrances. Wet hair is often tangled and difficult to comb following shampooing and for this reason it is common to apply rinses and conditioners in order to enhance ease of combing, detangling, body, shine, texture, prevention of static buildup and manageability. Creme rinses and combination creme rinse conditioners also generally improve the finish of hair.

Fixatives are designed to provide a temporary setting effect or curl to the hair and while the most common fixative is a hair spray which is designed to be applied to the hair after the hair has been blow dried, several specialty type fixatives can be applied either after the hair is towel dried or to dry hair, in order to provide more body and volume to the hair and to aid in styling, modeling and sculpting of the hair into unique new looks. This is followed by application of a hair spray in the form of an aerosol or pump spray to maintain the shape and style of the hair and provide gloss and sheen to the hair, in addition to a well groomed and natural appearance. Such specialty type fixatives are marketed under various names including styling gels, styling cremes, styling mousses, styling foams, styling sprays, styling spritz, styling mists, styling glazes, styling fixes; sculpting lotions, sculpting gels, sculpting glazes, sculpting sprays; glossing gels, glossing spritz; shaping gels; forming mousses; modeling spritz; finishing spritz; fixing gels; and setting lotions.

Whether the fixative is the more common hair spray or a specialty type fixative, it will typically include a film forming additive; a plasticizer or film modifying agent; a solvent which is usually an alcohol such as ethanol or a mixture of an alcohol and water; fragrances; and in the case of an aerosol spray, a propellant such as isobutane, butane, propane and blends thereof. The hair fixative should provide hair holding properties and curl retention, little flaking or powder on combing, rapid drying, nonstickiness, a lustrous feel without being oily and be easily removed by shampooing. The primary hair holding agent in any fixative such as a hair spray is the film forming material. The main function of the film forming material is to hold the hair in a styled configuration for a period of time. Exemplary film forming materials are shellac, polyvinylpyrrolidone, poly(vinylpyrrolidone-vinyl acetate), vinyl acetate-crotonic acid copolymer, methyl vinyl ether-maleic anhydride copolymer and octylacrylamide-acrylates-butylaminoethyl-methacrylate copolymer.

In accordance with the present invention, a new hair care formulation of any of the foregoing categories of hair care products is provided and which includes a high viscosity and high molecular weight organosilicon compound in the form of a mechanically prepared emulsion.

There are numerous processes known in the art for producing polysiloxane emulsions. These processes are categorized as being a mechanical emulsification process or an emulsion polymerization process. Emulsion polymerization entails taking a polysiloxane monomer and/or oligomer and emulsifying it in water in the presence of at least one surfactant while simultaneously polymerizing the monomer and/or oligomer in the presence of a catalyst to form the polysiloxane polymer. Emulsion polymerization processes have the capability to produce emulsions of high viscosity polysiloxanes, however, they may contain up to 20% or more of unreacted monomer or oligomer. The presence of monomer or oligomer is undesired in some applications using the emulsion. Processes known in the art for producing emulsions by emulsion polymerization are taught, for example, in U.S. Patent No. 2,891,920 to Hyde, U.S. Patent No. 3,294,725 to Findlay and U.S. Patent No. 3,360,491 to Axon.

Mechanical emulsification processes typically entail taking a polysiloxane polymer and emulsifying it in water in the presence of at least one surfactant using mechanical means such as agitation, shaking and homogenization, to achieve the emulsification. The ability to emulsify high viscosity polysiloxane polymers is limited when using mechanical emulsification processes. However, processes for mechanical emulsification are taught, for example, in U.S. Patent No. 2,702,276 to Green, U.S. Patent No. 2,755,194 to Volkmann, U.S. Patent No. 4,177,177 to Vanderhoff, U.S. Patent No. 4,620,878 to Gee and British Patent No. 2,000,798 to Schneider.

U.S. Patent No. 4,874,547, issued October 17, 1989, teaches a process for emulsifying silicones or mixture of silicones having a viscosity of less than 50 pascal-seconds (50,000 centipoise). The process involves the use of two different nonionic surfactants but not cationic or anionic surfactants. A rough emulsion is formed by mixing the silicone and a primary nonionic surfactant having an HLB value of 7 to 9. This mixture is further mixed with a secondary nonionic surfactant having an HLB value of 13 to 15 and water. The rough emulsion is subjected to low shear until an emulsion having a silicone particle size of less than about 2 microns is formed.

EPO Patent No. 200916 relates to a process for emulsifying silicones or mixture of silicones having a

viscosity of at least 50 pascal-seconds (50,000 centipoise). The process involves the use of three different nonionic surfactants but not cationic or anionic surfactants. The process requires mixing silicone and water in the presence of a primary nonionic surfactant having an HLB value of 13 to 15, a secondary nonionic surfactant having an HLB value of 7 to 9 and a tertiary nonionic surfactant having an HLB value of at least 16. Standard emulsions having a silicone particle size greater than 300 nanometers are produced by this process.

In contrast, the emulsions of the present invention are made by a process which produces emulsions from high viscosity polysiloxane polymers using mechanical means. In particular, the instant mechanical emulsification process produces emulsions from polysiloxane polymers having a viscosity of greater than 1,000 centistoke and preferably greater than 10,000 centistoke. The method produces oil-in-water emulsions from mixtures of polysiloxanes of at least one low viscosity polysiloxane and at least one high viscosity, non-volatile polysiloxane, including functional silicones and the emulsions have an average silicone particle size of less than about 350 nanometers.

The method produces oil-in-water emulsions from high viscosity polysiloxanes; mixtures of polysiloxanes referred to as bi-modal polysiloxane fluids of at least one low viscosity polysiloxane and at least one high viscosity non-volatile polysiloxane; functional polysiloxanes and mixtures thereof. Although useful for producing emulsions from high viscosity polysiloxanes, the method is also useful in producing emulsions from bi-modal polysiloxane fluids which include at least one low viscosity polysiloxane and at least one high viscosity non-volatile polysiloxane; and functional polysiloxanes such as amine functional polysiloxanes, carboxy functional polysiloxanes, haloalkyl functional polysiloxanes, glycol functional polysiloxanes and vinyl functional polysiloxanes.

The process is carried out by forming a thick phase emulsion from a polysiloxane, at least one primary nonionic surfactant having an HLB value of 10 to 19 and water. It may be necessary to combine the primary surfactant with sufficient water so that at the process temperature the surfactant is in a liquid state. After the primary surfactant and water have been dispersed into the polysiloxane, at least one secondary surfactant such as a nonionic, cationic or anionic surfactant having an HLB value of 1.8 to 15, is added and dispersed into the polysiloxane. The mixture is sheared at a temperature of 20°C. to 100°C. and preferably 40°C. to 100°C. for a sufficient time until the average silicone particle size of the thick phase emulsion is less than about 350 nanometers (nm), preferably less than 300 nanometers. The thick phase emulsion is diluted to any desired silicone content to form the final emulsion.

The high viscosity polysiloxanes are compounds having the formula

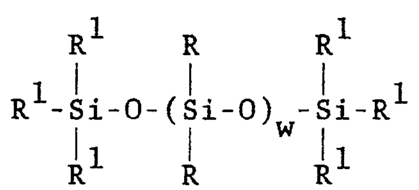

(I)

wherein R is hydrogen, an alkyl group having at least one carbon atom or an aryl group having 6 to 10 carbon atoms; $R^1$ is R, a hydroxy group or an alkoxy group; and w has a value such that the viscosity of the siloxane is at least 1,000 centistoke, preferably at least 10,000 centistoke. R may be methyl, ethyl, phenyl, vinyl or hydrogen. $R^1$ may be methyl, ethyl, vinyl, phenyl, -OH, methoxy, ethoxy or hydrogen.

The bi-modal polysiloxane fluids include at least one low viscosity polysiloxane and at least one high viscosity non-volatile polysiloxane. The low viscosity polysiloxane may be a linear or cyclic polysiloxane and may or may not be volatile. The low viscosity polysiloxanes are defined as those having a viscosity of less than 1,000 centistoke, preferably less than 500 centistoke. The low viscosity polysiloxane typically comprises 1 to 99% by weight of the bi-modal polysiloxane fluid composition.

The linear low viscosity polysiloxanes have the formula

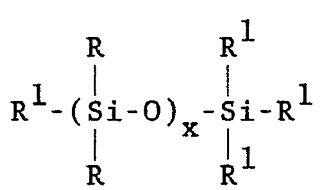

(II)

wherein R and $R^1$ are as described above and x has a value of at least one. R and $R^1$ should be predominantly methyl groups.

The cyclic low viscosity polysiloxanes have the formula

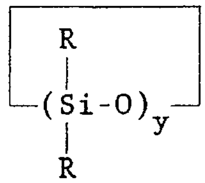

(III)

wherein R is described above and y has a value of 3 to 7.

Preferred compounds described by formula (I) have a viscosity of at least 10,000 centistoke and where R is predominantly methyl. The high viscosity non-volatile polysiloxane typically comprises 1 to 99% by weight of the bi-modal polysiloxane fluid composition.

The functional polysiloxanes are compounds having the formula

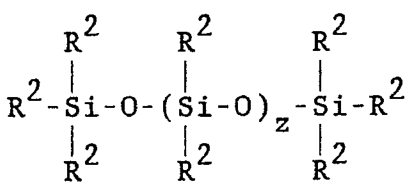

(IV)

wherein $R^2$ is $R^1$, aminoalkyl functional groups of at least one carbon atom, carboxyalkyl functional groups of at least one carbon atom, haloalkyl functional groups of at least one carbon atom, acrylate functional groups, acryloxy functional groups, acrylamide functional groups, vinyl functional groups and functional groups having the formula $F\text{-}R^3\text{-}$ where F is a functional group containing at least one atom which is not carbon or hydrogen and $R^3$ is an alkylene group having at least one carbon atom or an arylene group having 6 to 10 carbon atoms; and z has a value of at least 10. Where $R^2$ is a group other than methyl, the siloxane should contain less than 50% $R^2$ groups.

$R^2$ is exemplified by but not limited to the following functional groups:

$ClCH_2CH_2CH_2$-,

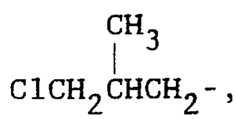

$H_2NCH_2CH_2NHCH_2CH_2CH_2$-,

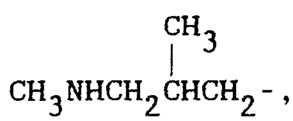

$F_3CCH_2CH_2$-,

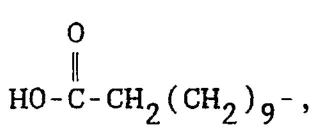

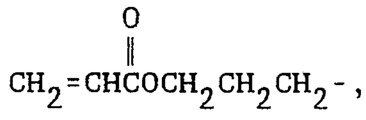

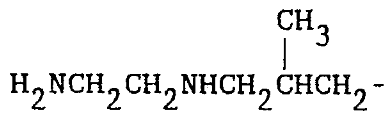

$HO(CH_2CH_2O)_{1\text{-}25}CH_2CH_2CH_2$-, and
$HO(CH_2CH_2O)_{1\text{-}25}(CH_2CH_2CH_2O)_{1\text{-}25}CH_2CH_2CH_2$-.

The primary surfactant is a nonionic surfactant having an HLB value of 10 to 19, such as polyoxyalklene alkyl ethers, polyoxyalkylene sorbitan esters, polyoxyalkylene esters or polyoxyalkylene alkylphenyl ethers. Representative primary surfactants are TERGITOL® TMN-6, TERGITOL® 15S40 and TERGITOL® 15S7 produced by Union Carbide Industrial Chemicals Division, Danbury, Connecticut, BRIJ 35 produced by ICI Americas, Inc, Willmington, Delaware and TRITON® X405 produced by Rohm and Haas Co., Philadelphia, Pennsylvania.

The secondary surfactant is a nonionic, cationic or anionic surfactant having an HLB value from 1.8 to 15. Useful nonionic surfactants are polyoxyalkylene alkyl ethers, polyoxyalkylene sorbitan esters, polyoxyalkylene esters or polyoxyalkylene alkylphenyl ethers. Representative nonionic secondary surfactants are BRIJ 30, TERGITOL® 15S5, TERGITOL® 15S3 and TERGITOL® TMN-3.

Suitable cationic surfactants are aliphatic fatty amines and derivatives such as dodecylamine acetate, octadecylamine acetate and acetates of the amines of tallow fatty acids; homologues of aromatic amines having fatty chains such as dodecylanalin; fatty amides derived from aliphatic diamines such as undecylimidazoline; fatty amides derived from disubstituted amines such as oleylaminodiethylamine; derivatives of ethylene diamine; quaternary ammonium compounds such as tallow trimethyl ammonium chloride, dioctadecyldimethyl ammonium chloride, didodecyldimethyl ammonium chloride and dihexadecyldimethyl ammonium chloride; amide derivatives of amino alcohols such as betahydroxyethylsterarylamide; amine salts of long chain fatty acids; quaternary ammonium bases derived from fatty amides of di-substituted diamines such as oleylbensylaminoethylene diethylamine hydrochloride; quaternary ammonium bases of the benzimidazolines such as methylheptadecyl benzimidazol hydrobromide; basic compounds of pyridinium and derivatives such as cetylpyridinium chloride; sulfonium compounds such as octadecylsulfonium methyl sulfate; quaternary ammonium compounds of betaine compounds of diethylamino acetic acid and octadecylchloromethyl ether; urethanes of ethylene diamine such as condensation products of stearic acid and diethylenetriamine; polyethylenediamines; and polypropanol polyethanol amines. Representative cationic surfactants are ARQUAD T27W, ARQUAD 16-29, ARQUAD C-33, ARQUAD T50 and ETHOQUAD T/13 ACETATE, manufactured by Akzo Chemical Division, Chicago, Illinois.

Suitable anionic surfactants are sulfonic acids and salt derivatives, including alkali metal sulforicinates; sulfonated glyceryl esters of fatty acids such as sulfonated monoglycerides of coconut oil acids; salts of sulfonated monovalent alcohol esters such as sodium oleylisethionate; amides of aminosulfonic acids such as the sodium salt of oleylmethyltauride; sulfonated products of fatty acid nitriles such as palmitonitrile sulfonate; sulfonated aromatic hydrocarbons such as sodium alpha-naphthalene monosulfonate; condensation products of naphthalene sulfonic acids with formaldehyde; sodium octahydroanthracene sulfonate; alkali metal alkylsulfates, ether sulfates having alkyl groups of 8 or more carbon atoms and alkylarylsulfonates having one or more alkyl groups of 8 or more carbon atoms.

The thick phase emulsions include at least 5 parts water per 100 parts of polysiloxane. The maximum amount of water should not exceed more than 20 parts of water per 100 parts of polysiloxane, however, the maximum amount of water is dependent upon the polysiloxane being emulsified. Further, the thick phase emulsions include one to 15 parts primary surfactant per 100 parts of polysiloxane and 1 to 15 parts secondary surfactant per 100 parts of polysiloxane. A preferred thick phase emulsion composition includes 5.5 to 12 parts water per 100 parts polysiloxane, 4 to 10 parts primary surfactant per 100 parts of polysiloxane and 4 to 10 parts secondary surfactant per 100 parts of polysiloxane.

The thick phase emulsion is diluted to the desired silicone content after an average silicone particle size of less than 350 nanometers, preferably less than 300 nanometers, has been achieved to produce the final emulsion. The thick phase emulsions can be diluted to one to 60 percent silicone content, typically 10 to 50 percent.

Optional ingredients which may be added to the final emulsion include preservatives, fungicides, antioxidants, rust and corrosion inhibitors and additional surfactants.

The process for producing the thick phase emulsion involves combining with shearing means a high

molecular weight polysiloxane, bi-modal polysiloxane fluids, functional polysiloxanes and mixtures thereof; at least one primary surfactant and water. The water is combined with the primary surfactant in an amount such that the primary surfactant is in the liquid state. It is preferred that the primary surfactant and water be dispersed in the polysiloxane at a temperature between 20° C. and 100° C., more preferably between 40° C. and 100° C. After the primary surfactant and water have been dispersed, at least one secondary surfactant is added and dispersed into the polysiloxane using shear means. The resulting composition is heated and maintained at a temperature between 20° C. and 100° C., preferably between 40° C. and 100° C. and mixed using shearing means for a sufficient period of time until the average silicone particle size is less than 350 nanometers, more preferably less than 300 nanometers. The resulting thick phase emulsion is diluted with additional water to a desired silicone content to form a final emulsion.

The final emulsions are stable and oil-free. A stable emulsion is defined by no visible signs of creaming or free-oil in the emulsion after one month at room temperature. An oil-free emulsion is defined as an emulsion that does not have visible signs of unemulsified silicone oil after processing is complete.

It is preferred that the process including the preliminary mixing be carried out at temperatures between 20° C. and 100° C., more preferably 40° C. and 100° C. and at atmospheric pressure. Heat can be applied by shear forces, electrical means, steam or hot oil.

The components of the emulsion are mixed and emulsified using shearing means such as single screw extruders, multiple screw extruders, change can mixers, planetary mixers, kettle mixers, static mixers, blend tanks, paddle mixers, butterfly mixers, sigma blade mixers, G-force mixers, mill type mixers, turbulizer mixers, finger mixers, porcupine mixers and helicone mixers. Any shear generating means which produce the necessary shear to form the thick phase emulsions can be used. The mixing and emulsification process can be carried out as a batch or continuous process depending on the shearing means used.

The time required to produce a thick phase emulsion having an average silicone particle size of less than 350 nanometers is dependent upon the amount of shear, the amount and type of secondary surfactant, the water concentration and the temperature during processing. Thick phase emulsions with an average silicone particle size of less than 350 nanometers can be provided in less than 24 hours, typically in less than 6 hours. It is important to shear the thick phase emulsion composition for a sufficient period of time until the average silicone particle size is less than 350 nanometers, preferably less than 300 nanometers.

The following examples illustrate preparation of the mechanical emulsions. The average silicone particle size of the emulsions was measured with a Nicomp Submicron Particle Sizer, Model 370 of Pacific Scientific, Silver Spring, Md. The average particle size is reported as the "intensity weighted" value as determined by quasi-elastic light scattering and the cumulant method of D. E. Koppel, Journal of Chem. Phys., 57, page 4814, 1972. The term parts used herein refers to parts by weight.

Example 1

88.5 parts of a 60,000 cSt polydimethylsiloxane fluid was charged to a clean Turello mixer with a double sweep action blade. The fluid was heated to approximately 60° C. by the shearing action of the mixer blades. 4.3 parts of BRIJ-35 surfactant having an HLB of 16.9 dissolved in 5.1 parts of water was added. After approximately 10 minutes of continued mixing, 5.1 parts of BRIJ-30 surfactant having an HLB of 9.7, was added and the mixing was continued for 4 hours at 60° C. The resulting average silicone particle size was 289 nanometers and there was no visible signs of unemulsified polysiloxane. The thick phase emulsion was diluted to 50% silicone. The final emulsion did not cream after three months at room temperature and at 40° C. and was stable through five freeze/thaw cycles.

Example I-A

50 parts of a bi-modal polysiloxane fluid including 13% of a hydroxy endblocked high molecular weight polydimethylsiloxane in a mixture of octamethyltetracyclosiloxane and decamethylpentacyclosiloxane was charged to a clean Turello mixer. The bi-modal polysiloxane fluid was heated to approximately 60° C. by a combination of steam in the mixer jacket and shearing action of the mixer blades. 2.5 parts of BRIJ-35 dissolved in 6 parts of water was added and mixed until uniform. 3.5 parts of BRIJ-30 was added and mixing was continued for 4 hours at 55° C. The mixture was cooled and diluted with 38 parts additional water. The final emulsion was oil-free and had an average silicone particle size of 269 nanometers. After 4 months at room temperature, the final emulsion did not cream.

Example I-B

35 parts of a bi-modal polysiloxane fluid including 13% of a hydroxyendblocked high molecular weight polydimethylsiloxane in a mixture made up of octamethyltetracyclosiloxane and decamethylpentacyclosiloxane was charged to a clean Turello mixer. The bi-modal polysiloxane fluid was heated to approximately 60° C. by the shearing action of the mixer blades. 2.4 parts of BRIJ-35 dissolved in 4 parts of water was added and mixed until uniform. 2.4 parts of BRIJ-30 was added and mixing was continued for 3 hours. The mixture was cooled and diluted with 56.2 parts additional water. The final emulsion was oil-free and had an average particle size of 267 nanometers. After 4 months at room temperature, the final emulsion did not cream.

Example I-C

50 parts of a bi-modal polysiloxane fluid including 13% of a hydroxyendblocked high molecular weight polydimethylsiloxane, a mixture of dimethylcyclicsiloxanes and 5 cSt polydimethylsiloxane fluid, was charged to a clean Turello mixer. The bi-modal polysiloxane fluid was heated to approximately 60° C. by a combination of steam in the mixer jacket and shearing action of the mixer blades. 2.5 parts of BRIJ-35 dissolved in 1 part of water was added and mixed until uniform. 4 parts of additional water was added and mixed until uniform. 3.5 parts of BRIJ-30 was added and mixing was continued for 3 hours at 64° C. The mixture was cooled and diluted with 39 parts additional water. The final emulsion was oil-free and had an average particle size of 222 nanometers. After 2 months at room temperature, the final emulsion did not cream.

Example II

50 parts of a hydroxyendblocked polytrifluoropropylmethylsiloxane fluid with a viscosity of approximately 50,000 cSt was charged to a clean Turello mixer. The siloxane fluid was heated to approximately 60° C. by the shearing action of the mixer blades. 2.5 parts of BRIJ-35 dissolved in one part of water was added followed by 2.5 parts of additional water. After mixing until uniform, 3.5 parts of BRIJ-30 was added and mixing was continued for 3 hours. The mixture was cooled and diluted with 40.5 parts additional water. The final emulsion was oil-free and had an average particle size of 269 nanometers.

Example II-A

35 parts of a bi-modal polysiloxane fluid including 13% of a hydroxyendblocked high molecular weight polydimethylsiloxane in a mixture of octamethyltetracyclosiloxane and decamethylpentacyclosiloxane was charged to a clean Turello mixer. The bi-modal polysiloxane fluid was heated to approximately 60° C. by the shearing action of the mixer blades. 2.2 parts of TERGITOL® 15S7 having an HLB of 12.2 and 3.6 parts of water were added and mixed until uniform. 0.55 parts of ARQUAD T27W cationic surfactant was added and mixing was continued for 3 hours. The mixture was cooled and diluted to 35% silicone. The final emulsion was oil-free and had an average particle size of 338 nanometers. After 4 months at room temperature, the final emulsion did not cream.

Additional examples to illustrate the concepts of the present invention are set forth below.

Example III

Into a vessel was placed 332 g deionized water, 15.45 g of coconut fatty acid diethanolamide and 150.23 g of a 30% aqueous solution of ammonium lauryl sulfate. The contents were mixed until a clear, homogenous solution was obtained.

Example IV

Into a vessel was placed 15.0 g of the solution prepared in Example III. Aqueous citric acid (20%) was added to adjust the pH to 5.5. Ammonium chloride, 0.09 g, was added to provide a final shampoo viscosity of 3000 cS. This shampoo was employed as a comparative formulation in the remaining examples and tests which follow.

Table I shows the particulars of the silicon emulsions used to produce the shampoo formulations of Examples V-XIII.

TABLE I

| Silicone Emulsion | Description of Silicone |
|---|---|
| A | Blend of high viscosity dimethyl siloxane gum and 350 cS dimethyl siloxane fluid |
| B | Blend of high viscosity dimethyl siloxane gum and cyclodimethyl siloxanes |
| C | Blend of high viscosity dimethyl siloxane gum and 5 cS dimethyl siloxane fluid |
| D | Blend of high viscosity trimethylsilylamodimethicone fluid and cyclodimethylsiloxanes |
| E | Blend of low viscosity trimethylsilylamodimethicone fluid and cyclodimethylsiloxanes |
| F | Blend of high viscosity dimethyl siloxane gum and phenyl trimethylsiloxy siloxanes |
| G | A trifluoropropyl methyl siloxane fluid |
| H | A 60,000 cS polydimethylsiloxane fluid |
| I | Blend of high viscosity dimethyl siloxane gum and cyclodimethyl siloxanes |

The following examples illustrate the preparation of shampoos in accordance with the present invention employing various of the silicon emulsions shown in Table I.

Example V

Into a vessel was placed 15.0 g of the solution prepared in Example III. Also added was 0.9 g of silicone emulsion A. Aqueous citric acid (20%) was added to adjust the pH to 5.5. Ammonium chloride, 0.12 g, was added to provide a final shampoo viscosity of 2104 cS. A pearlescent conditioning shampoo was obtained.

Example VI

Into a vessel was placed 15.0 g of the solution prepared in Example III. Also added was 0.9 g of silicone emulsion B. Aqueous citric acid (20%) was added to adjust the pH to 5.5. Ammonium chloride, 0.12 g, was added to provide a pearlescent conditioning shampoo.

Example VII

Into a vessel was placed 15.0 g of the solution prepared in Example III. Also added was 0.9 g of silicone emulsion C. Aqueous citric acid (20%) was added to provide a pearlescent conditioning shampoo with a viscosity of 142 cS.

Example VIII

Into a vessel was placed 15.0 g of the solution prepared in Example III. Also added was 0.9 g of silicone emulsion D. Aqueous citric acid (20%) was added to adjust the pH to 5.5. Ammonium chloride, 0.12 g, was added to provide a pearlescent conditioning shampoo.

Example IX

Into a vessel was placed 15.0 g of the solution prepared in Example III. Also added was 0.9 g of silicone emulsion E. Aqueous citric acid (20%) was added to adjust the pH to 5.5. Ammonium chloride, 0.09 g, was added to provide a pearlescent mixture.

Example X

Into a vessel was placed 15.0 g of the solution prepared in Example III. Also added was 0.9 g of silicone emulsion F. Aqueous citric acid (20%) was added to adjust the pH to 5.5. Ammonium chloride, 0.12 g, was added to provide a pearlescent conditioning shampoo.

Example XI

Into a vessel was placed 15.0 g of the solution prepared in Example III. Also added was 0.9 g of silicone emulsion G. Aqueous citric acid (20%) was added to adjust the pH to 5.5. Ammonium chloride, 0.12 g, was added to provide a final shampoo viscosity of 2780 cS. A pearlescent conditioning shampoo was obtained.

Example XII

Into a vessel was placed 15.0 g of the solution prepared in Example III. Also added was 0.9 g of silicone emulsion H. Aqueous citric acid (20%) was added to adjust the pH to 5.5. Ammonium chloride, 0.09 g, was added to provide a final shampoo viscosity of 1813 cS. A pearlescent conditioning shampoo was obtained.

Example XIII

Into a vessel was placed 15.0 g of the solution prepared in Example III. Also added was 0.9 g of silicone emulsion I. Aqueous citric acid (20%) was added to adjust the pH to 5.5. Ammonium chloride, 0.12 g, was added to provide a pearlescent conditioning shampoo.

The above formulations were tested as shampoos on dark European hair swatches and compared to dark European hair swatches which had been treated with the comparative shampoo of Example IV, on the basis of ease of wet and dry combing. The combing rating scale ranged form one to five, with one being easy to comb and offering very little resistance and five being impossible to move the comb through the hair. The testing procedure employed involved shampooing the swatches with the comparative shampoo of Example IV and drying the test swatches. About one-half gram of the shampoo formulation of the present invention was applied to the test swatch after the swatch had been rewetted. The shampoo formulation was worked into the swatch for about twenty seconds and the swatch was rinsed for about thirty seconds. The swatch was detangled by passing the wide portion of a comb through the swatch once. The swatch was hung to dry and evaluated at the end of twenty-four hours. The test results are shown in Table II and indicate that all of the shampoo formulations of the present invention which were tested evidenced combing improvements in comparison to the formulation of Example IV which contained no silicone ingredient.

TABLE II

| EXAMPLE | DRY COMBING | WET COMBING |
|---|---|---|
| IV | 3 | 2.75 |
| V | 2.5 | 2 |
| VI | 2.5 | 2 |
| VII | 2.5 | 2 |
| VIII | 2 | 2 |
| X | 2 | 2 |
| XI | 1.5 | 2.5 |
| XII | 2 | 2.5 |
| XIII | 2 | 2 |

Following are additional examples similar to Examples V-XIII but showing the preparation of thickened and/or stabilized shampoo formulations relative to shampoos of Examples V-XIII.

Example XIV

Into a vessel was placed 16.0 g of the mixture prepared in Example VI and 3.2 g of a 10% aqueous solution of PEG-120 methyl glucose dioleate which is a methyl glucoside derivative with two polyoxyethylene substituent groups containing a total of 120 oxyethylene units on the average. In addition, 0.09 g of ammonium chloride was added to provide a pearlescent conditioning shampoo.

Example XV

Into a vessel was placed 16.0 g of the mixture prepared in Example VIII and 3.2 g of a 10% aqueous solution of PEG-120 methyl glucose dioleate. In addition, 0.09 g of ammonium chloride was added to provide a pearlescent conditioning shampoo.

Example XVI

Into a vessel was placed 16.0 g of the mixture prepared in Example X and 3.2 g of a 10% aqueous solution of PEG-120 methyl glucose dioleate. In addition, 0.09 g of ammonium chloride was added to provide a pearlescent conditioning shampoo.

Example XVII

Into a vessel was placed 16.0 g of the mixture prepared in Example XIII and 3.2 g of a 10% aqueous solution of PEG-120 methyl glucose dioleate. In addition, 0.09 g of ammonium chloride was added to provide a pearlescent conditioning shampoo.

The thickened and/or stabilized shampoos of Examples XIV-XVII were tested as noted previously and the results are shown in Table III. These shampoos provided improved combing in comparison to the comparative shampoo of Example IV which contained no silicone ingredient.

TABLE III

| EXAMPLE | DRY COMBING | WET COMBING |
|---|---|---|
| IV | 2.5 | 3.5 |
| XIV | 1.5 | 2.5 |
| XV | 2 | 2 |
| XVI | 2 | 1.75 |
| XVII | 1.5 | 2 |

Example XVIII

Into a vessel was placed 16.0 g of the mixture prepared in Example V and 2 g of a 10% aqueous solution of PEG-120 methyl glucose dioleate. In addition, 0.28 g of ammonium chloride 4.8 g of a 30% aqueous solution of ammonium lauryl sulfate, 0.3 g coco acid DEA and sufficient quantity of 20% aqueous citric acid to give a final pH of 5.5 were added to provide a pearlescent conditioning shampoo with a viscosity of 835 cS.

Example XIX

Into a vessel was placed 16.0 g of the mixture prepared in Example XI and 2 g of a 10% aqueous solution of PEG-120 methyl glucose dioleate. In addition, 0.30 g of ammonium chloride 4.8 g of 30% aqueous solution of ammonium lauryl sulfate, 0.5 g coco acid DEA and 20% aqueous citric acid to give a final pH of 5.5 were added to provide a pearlescent conditioning shampoo with a viscosity of 721 cS.

Example XX

Into a vessel was placed 16.0 g of the mixture prepared in Example XII and 2 g of a 10% aqueous solution of PEG-120 methyl glucose dioleate. In addition, 0.32 g of ammonium chloride, 4.8 g of 30% aqueous solution of ammonium lauryl sulfate, 0.3 g of coco acid DEA and 20% citric acid to give a final pH of 5.5 were added to provide a pearlescent conditioning shampoo with a viscosity of 752 cS.

The thickened and/or stabilized shampoos of Examples XVIII-XX were tested as noted previously and the results are shown in Table IV. These shampoos provided improved combing in comparison to the comparative shampoo of Example IV which contained no silicone ingredient.

TABLE IV

| EXAMPLE | DRY COMBING | WET COMBING |
|---|---|---|
| IV | 2.75 | 4.0 |
| XVIII | 2 | 2.25 |
| XIX | 2 | 2.5 |
| XX | 1.75 | 1.5 |

The compositions of this invention may contain a surfactant selected from the group consisting of anionic and amphoteric surfactants. The surfactant system should provide an acceptable level of foam on the hair and be capable of cleaning the hair and may comprise one or more water soluble detergents, i.e., an anionic or amphoteric surfactant. Suitable anionic detergents include sulfonated and sulfated alkyl, aralkyl and alkaryl anionic detergents; alkyl succinates; alkyl sulfosuccinates and N-alkyl sarcosinates. Especially preferred are the sodium, magnesium, ammonium and the mono-, di- and triethanolamine salts of alkyl and aralkyl sulfates as well as the salts of alkaryl sulfonates. The alkyl groups of the detergents generally have a total of from about 12 to 21 carbon atoms, may be unsaturated and are preferably fatty alkyl groups. The sulfates may be sulfate ethers containing one to ten ethylene oxide or propylene oxide units per molecule. Preferably, the sulfate ethers contain 2 to 3 ethylene oxide units.

Typical anionic detergents include, among others, sodium lauryl sulfate, sodium lauryl ether sulfate, ammonium lauryl sulfate, triethanolamine lauryl sulfate, sodium C14-16 olefin sulfonate, ammonium pareth-25 sulfate (ammonium salt of a sulfated polyethylene glycol ether of a mixture of synthetic C12-15 fatty alcohols), sodium myristyl ether sulfate, ammonium lauryl ether sulfate, disodium mono-oleamidosulfosuccinate, ammonium lauryl sulfosuccinate, sodium dodecylbenzene sulfonate, triethanolamine dodecylbenzene sulfonate and sodium N-lauroyl sarcosinate. The most preferred anionic detergents are the lauryl sulfates, particularly monoethanolamine, triethanolamine, ammonium and sodium lauryl sulfates. Sodium lauryl ether sulfate is also very suitable for use in the compositions of this invention.

Surfactants generally classified as amphoteric or ampholytic detergents include, among others, cocoamphocarboxyglycinate, cocoamphocarboxypropionate, cocobetaine, N-cocamidopropyldimethylglycine and N-lauryl-N-carboxymethyl-N-(2-hydroxyethyl)ethylenediamine. Other suitable amphoteric detergents include the quaternary cycloimidates, betaines and sultaines disclosed in U.S. Patent No. 3,964,500. Particularly preferred amphoteric surfactants are the substituted quaternary hydroxy cycloimidinic acid alkali

metal alcoholates described in U.S. Patent No. 2,528,378.

The most preferred of the amphoteric surfactants are the substituted quaternary hydroxy cycloimidinic acid alkali metal alkoxymethyl carboxylates described in U.S. Patent No. 2,781,354. The betaines may have the structure:

$R^1R^2R^3N^+(CH_2)_mCOO^-$

wherein $R^1$ is an alkyl group having about 12 to 18 carbon atoms or a mixture thereof, $R^2$ and $R^3$ are independently lower alkyl groups having 1 to 3 carbon atoms and m is an integer from 1 to 4. Specific betaines useful in the products of the invention are for example alpha-(tetradecyldimethylammonio)acetate, beta-(hexadecyldiethylammonio)propionate and gamma-(dodecyldimethylammonio)butyrate.

The sultaines may have the structure:

$R^1R^2R^3N^+(CH_2)_mSO_3^-$

wherein $R^1$, $R^2$, $R^3$ and m are defined as above. Specific useful sultaines are for example 3-(dodecyldimethylammonio)propane-1-sulfonate and 3-(tetradecyldimethylammonio)ethane-1-sulfonate.

The compositions of this invention may contain a nonionic surfactant. The nonionic surfactants of the present invention are selected from the group consisting of fatty acid alkanolamide and amine oxide surfactants. The fatty acid alkanolamides are nonionic surfactants obtained by reacting alkanolamines such as monoethanolamine, diethanolamine, monoisopropanolamine or diisopropanolamine with a fatty acid or fatty acid ester to form the amide. The hydrophobic portion of the nonionic surfactant is provided by a fatty acid hydrocarbon chain which generally has from 10 to 21 carbon atoms. The fatty acid alkanolamide surfactants include, for example, fatty acid diethanolamides such as isostearic acid diethanolamide, lauric acid diethanolamide, capric acid diethanolamide, coconut fatty acid diethanolamide, linoleic acid diethanolamides, myristic acid diethanolamide, oleic acid diethanolamide and stearic acid diethanolamide; fatty acid monoethanolamides such as coconut fatty acid monoethanolamide; and fatty acid monoisopropanolamides such as oleic acid monoisopropanolamide and lauric acid monoisopropanolamide.

The amine oxides are well known nonionic surfactants usually obtained by oxidizing a tertiary amine to form the amine oxide. They are sometimes also referred to as polar nonionic surfactants. Amine oxide surfactants include, for example, the N-alkyl amine oxides such as N-cocodimethylamine oxide, N-lauryl dimethylamine oxide, N-myristyl dimethylamine oxide and N-stearyl dimethylamine oxide; the N-acyl amine oxides such as N-cocamidopropyl dimethylamine oxide and N-tallowamidopropyl dimethylamine oxide and N-alkoxyalkyl amine oxides such as bis(2-hydroxyethyl) $C_{12-15}$ alkoxy-propylamine oxide. The hydrophobic portion of the amine oxide surfactants is generally provided by a fatty hydrocarbon chain containing from 10 to 21 carbon atoms.

For purposes of this invention, the alkanolamide and amine oxide surfactants are preferred. In general, the fatty acid diethanolamides and N-alkyl dimethylamine oxides are preferred for use in the compositions. Especially preferred are the fatty acid diethanolamides and N-alkyl dimethylamine oxides where the fatty hydrocarbon chain contains from 10 to 18 carbon atoms. For example, especially preferred nonionic surfactants include lauric acid diethanolamide, N-lauryl dimethylamine oxide, coconut acid diethanolamide, myristic acid diethanolamide and oleic acid diethanolamide.

Additional categories of surfactant materials may also be included such as cationic and zwitterionic surfactants and representative compounds are set forth in detail in U.S. Patent No. 4,902,499, issued February 20, 1990.

Other adjuvants may be added to the compositions of this invention such as thickeners, perfumes, colorants, electrolytes, pH control ingredients, antimicrobials, antioxidants, ultraviolet light absorbers and medicaments. For example, it is sometimes preferred to employ a thickener in the compositions to facilitate the hand application of the composition to the hair. Thickeners are preferably used in sufficient quantities to provide a convenient viscosity. For example, viscosities within the range of 400 to 6000 cps or more preferably in the range of 1000 to 4000 cps as measured at 25° C. are usually suitable.

Suitable thickeners, include, among others, sodium alginate, gum arabic, polyoxyethylene, guar gum, hydroxypropyl guar gum, cellulose derivatives such as methylcellulose, methylhydroxypropylcellulose, hydroxypropylcellulose, polypropylhydroxyethylcellulose, starch and starch derivatives such as hydroxyethylamylose and starch amylose, locust bean gum, electrolytes such as NaCl, saccharides such as fructose and glucose and derivatives of saccharides such as PEG-120 methyl glucose dioleate. Preferred thickeners include the cellulose derivatives and saccharide derivatives. The glucose derivative, PEG-120 methyl glucose dioleate, is especially preferred in the present invention.

The perfumes which can be used in the compositions are the cosmetically acceptable perfumes. Colorants are used to confer a color to the composition and may generally be used. Although not required, it is preferred to employ an acid to adjust the pH within the range of 5 to 9 or more preferably within the range of 6 to 8 in the compositions of this invention. Any water soluble acid such as a carboxylic acid or a mineral acid is suitable. For example, suitable acids include mineral acids such as hydrochloric, sulfuric and phosphoric, monocarboxylic acids such as acetic acid, lactic acid or propionic acid; and polycarboxylic acids such as succinic acid, adipic acid and citric acid.

If for special purposes conditioners are desired, they may be added. For example, any of the well-known organic cationic hair conditioning components may be added. Some cationic conditioning components that may be used in the present invention to provide hair grooming include quaternary nitrogen derivatives of cellulose ethers, homopolymers of dimethyldiallyl-ammonium chloride, copolymers of acrylamide and dimethyldiallylammonium chloride, homopolymers or copolymers derived from acrylic acid or methacrylic acid containing cationic nitrogen functional groups attached to the polymer via ester or amide linkages, polycondensation products of N,N'-bis-(2,3-epoxypropyl)-piperazine or of piperazine-bis-acrylamide and piperazine, poly(dimethylbutenylammonium chloride)-α,ω-bis-(triethanol-ammonium) chloride and copolymers of vinylpyrrolidone and acrylic acid esters with quaternary nitrogen functionality. The above cationic organic polymers and others are described in more detail in U.S. Patent No. 4,240,450. Other categories of conditioners may also be employed.

A preservative may be required and representative preservatives which may be employed include about 0.1-0.2 weight percent of compounds such as formaldehyde, dimethyloldimethylhydantoin, 5-bromo-5-nitro-1,3-dioxane, methyl and propyl para-hydroxybenzoates and mixtures of such benzoates with sodium dehydroacetate, sorbic acid and imidazolidinyl urea.

The compositions of the present invention may also be formulated to include dyes, colorants, reducing agents, neutralizing agents and preservatives, necessary for their application as permanent wave systems or hair dyes, for example. The active formulation can be applied in several different forms including lotions, gels, mousses, aerosols and pump sprays, for example, and as conditioners and shampoos. The active ingredient may include a carrier and suitable carrier fluids for hair care formulations are water as well as, for example, such fluids as alcohols namely ethanol or isopropanol, hydrocarbons and halogenated hydrocarbons as mineral spirits and trichloroethane, cyclic siloxanes and aerosol propellants.

When the composition is intended for aerosol application, propellant gases can be included such as carbon dioxide, nitrogen, nitrous oxide, volatile hydrocarbons such as butane, isobutane or propane and chlorinated or fluorinated hydrocarbons such as dichlorodifluoromethane and dichlorotetrafluoroethane or dimethylether.

It will be apparent from the foregoing that many other variations and modifications may be made in the compounds, compositions and methods, described herein, without departing substantially from the essential features and concepts of the present invention. Accordingly, it should be clearly understood that the forms of the invention described herein are exemplary only and are not intended as limitations on the scope of the present invention as defined in the appended claims.

## Claims

1. In a method of treating hair by applying to the hair a formulation including at least one organosilicon compound and at least one additive selected from the group consisting of surfactants, conditioners, thickeners, plasticizers, fragrances, propellants and preservatives, the improvement comprising the step of incorporating the organosilicon compound into the formulation in the form of a mechanically prepared emulsion, the particle size of the organosilicon compound in the emulsion being less than about 350 nanometers.

2. The method of claim 1 in which the organosilicon compound is selected from the group consisting of high viscosity polysiloxanes, high molecular weight polysiloxanes, functional polysiloxanes and bi-modal polysiloxanes and in which the organosilicon compound is a polysiloxane having a viscosity of at least 50,000 centistokes measured at 25° C.

3. The method of claim 2 in which the organosilicon compound is a functional polysiloxanes having a functional group selected from the group consisting of carboxy, carboxyalkyl, haloalkyl, glycol, acrylamide, acrylate, acryloxy, vinyl and amino groups.

4. In a composition for treating hair which includes at least one organosilicon compound and at least one

additive selected from the group consisting of surfactants, conditioners, thickeners, plasticizers, fragrances, propellants and preservatives, the improvement comprising employing as the organosilicon compound a mechanically prepared emulsion which contains the organosilicon compound, the particle size of the organosilicon compound in the emulsion being less than about 350 nanometers.

**5.** The composition of claim 4 in which the organosilicon compound is selected from the group consisting of high viscosity polysiloxanes, high molecular weight polysiloxanes, functional polysiloxanes and bimodal polysiloxanes and in which the organosilicon compound is a polysiloxane having a viscosity of at least 50,000 centistokes measured at 25° C.

**6.** The composition of claim 5 in which the organosilicon compound is a functional polysiloxanes having a functional group selected from the group consisting of carboxy, carboxyalkyl, haloalkyl, glycol, acrylamide, acrylate, acryloxy, vinyl and amino groups.

**7.** The composition of claim 5 in which the organosilicon compound is a bi-modal polysiloxane, the bimodal polysiloxane being a mixture of a nonvolatile polydimethylsiloxane and a polydimethylsiloxane selected from the group consisting of volatile and low viscosity polydimethylsiloxanes.